# DEMANDE DE BREVET EUROPEEN

(11) **EP 3 162 383 A1**
(43) Date de publication de la demande: **03.05.2017**
(21) Numéro de dépôt: 16193648.9
(22) Date de dépôt: 13.10.2016
(51) Int. Cl.: A61L 9/12, B60H 3/00

(54) **DISPOSITIF DE DIFFUSION D'AU MOINS UN AGENT VOLATIL**

(30) Priorité: 29.10.2015 FR 1560345
(71) Demandeur: Valeo Systemes Thermiques, 78320 Le Mesnil Saint Denis (FR)
(72) Inventeur: RIVET, Gilles, 28500 Charpont (FR)
(74) Mandataire: Metz, Gaëlle

(57) **Abrégé**

La présente invention concerne un dispositif de diffusion comprenant un ou plusieurs éléments de diffusion adaptés pour stocker et diffuser une quantité déterminée d'agents volatils, ledit dispositif étant pourvu d'un ou plusieurs canaux (2, 2') de circulation d'un flux d'air adaptés pour que ledit flux d'air se charge en agents volatils au contact desdits éléments de diffusion, ledit dispositif comprenant en outre un organe de commande (6), actionnable manuellement par un utilisateur, ledit organe de commande (6) étant configuré pour prendre différentes positions sous l'action de l'utilisateur, lesdites positions mettant ou non en communication le ou l'un desdits canaux (2, 2') de circulation d'air et un canal (5) de passage d'air dudit organe de commande (6).

## Description

La présente invention concerne un dispositif de diffusion d'au moins un agent volatil, notamment d'au moins une fragrance. Plus précisément, l'invention vise le domaine des véhicules automobiles et l'équipement de leur habitacle.

De nos jours, les constructeurs de véhicules automobiles souhaitent parfois intégrer un système permettant de diffuser un agent volatil dans l'habitacle desdits véhicules automobiles. Quand les véhicules n'en sont pas équipés de série, les utilisateurs peuvent de leur côté ressentir le besoin d'équiper leur véhicule d'un tel système.

De nombreuses solutions ont été proposées en ce sens. Quand il s'agit de systèmes intégrés au véhicule, les solutions proposées restent souvent complexes, notamment en prévoyant une commande à distance des composants du système contrôlant la diffusion. Quand il s'agit de systèmes rapportés par l'utilisateur du véhicule, il s'agit à l'inverse de solutions n'offrant pas de possibilité de contrôle de la diffusion ou alors selon des configurations dont le niveau d'intégration reste limité.

L'invention se propose de pallier les inconvénients précités et concerne à cette fin un dispositif de diffusion, notamment pour véhicules automobiles, comprenant un ou plusieurs éléments de diffusion adaptés pour stocker et diffuser une quantité déterminée d'agents volatils, ledit dispositif étant pourvu d'un ou plusieurs canaux de circulation d'un flux d'air adaptés pour que ledit flux d'air se charge en agents volatils au contact desdits éléments de diffusion.

Selon l'invention, ledit dispositif comprend en outre un organe de commande, actionnable manuellement par un utilisateur, ledit organe de commande étant configuré pour prendre différentes positions sous l'action de l'utilisateur, lesdites positions mettant ou non en communication le ou l'un desdits canaux de circulation d'air et un canal de passage d'air dudit organe de commande.

Autrement dit, en cas de canal de circulation d'air unique, ledit organe de commande permet, en fonction de sa position, de mettre en correspondance, partielle ou totale, ledit canal de circulation d'air avec le canal de passage d'air dudit organe de commande ou de fermer ledit canal de circulation d'air. Ledit organe de commande permet alors de faire un réglage en tout ou rien, voir un réglage de débit du flux d'air chargé en agents volatils.

En cas de pluralité de canaux de circulation d'air, selon un mode de réalisation préférentiel, ledit organe de commande permet, en fonction de sa position, de mettre en correspondance, partielle ou totale, l'un desdits canaux de circulation et ledit canal de passage d'air de l'organe de commande, les autres canaux de circulation étant fermés. On réalise de la sorte une fonction de sélection du flux d'air et donc de l'agent volatil diffusé, voire un réglage de débit.

Dans un tel mode, l'organe de commande pourra être configuré pour que l'une de ses positions au moins corresponde à une fermeture de l'ensemble des canaux de circulation d'air. En variante, l'une des positions de l'organe de commande pourra correspondre à la mise en communication du canal de passage avec un canal de circulation d'air ne provoquant pas une mise en contact du flux d'air avec l'un des agents volatils du dispositif.

En variante encore, l'organe de commande pourra comprendre plusieurs canaux de passage du flux d'air pour offrir une plus grande possibilité de débit et/ou des mélanges de flux d'air chargés de différents agents volatils.

Grâce à l'intégration d'un organe de commande dans le dispositif, on dispose d'une solution dont le fonctionnement est autonome. Le dispositif de diffusion conforme à l'invention peut de la sorte aussi bien être utilisé en tant qu'équipement de première monte qu'à titre de produit après-vente. En outre, grâce à l'intégration du conduit de passage d'air dans l'organe de commande lui-même, on obtient un dispositif présentant une grande compacité.

Selon différentes caractéristiques complémentaires, qui pourront être prises ensemble ou séparément :
- ledit dispositif comprend un ventilateur, apte à générer ledit flux d'air,
- ledit dispositif comprend un réservoir, contenant le ou lesdits éléments de diffusion, le ou lesdits canaux de circulation traversant ledit réservoir,
- l'organe de commande, le réservoir et/ou le ventilateur sont disposés de sorte qu'une direction d'un flux d'air circulant entre le ventilateur et le réservoir est alignée avec la direction du flux d'air circulant dans le ou les canaux de circulation du réservoir et/ou dans le canal de passage d'air dudit organe de commande,
- la trajectoire du flux d'air traversant l'organe de commande, le réservoir et/ou le ventilateur est sensiblement rectiligne,
- ledit organe de commande, ledit réservoir et/ou ledit ventilateur sont disposés l'un sur l'autre selon une direction d'empilement,
- ledit dispositif comprend un boîtier accueillant ledit ventilateur et ledit réservoir,
- ledit boitier est fermé par ledit organe de commande, prévu mobile par rapport audit boîtier,
- le réservoir est amovible par rapport audit boîtier,
- ledit boîtier comprend une extrémité, apte à être insérée dans un support dudit dispositif, par exemple un porte-gobelet,
- ledit boîtier comprend une ou des entrées d'air, positionnées de façon à être laissées libres une fois ledit dispositif inséré dans son support,
- ledit ventilateur est situé au niveau de ladite extrémité,
- la ou les entrées d'air sont disposées entre le ventilateur et une zone dudit boîtier accueillant ledit réservoir,
- le boîtier et/ou l'organe de commande comprennent des éléments d'indexation visuelle et/ou mécanique permettant de repérer la position relative dudit organe de commande par rapport au boîtier,
- ledit organe de commande est mobile en rotation autour d'un axe de rotation,
- ledit organe de commande comprend au moins un couvercle disposé sur le réservoir et pourvu dudit canal de passage du flux d'air,
- l'organe de commande comprend en outre au moins un disque muni d'une ouverture située en correspondance dudit canal de passage du flux d'air, ledit couvercle et ledit disque étant situés de part et d'autre dudit réservoir,ledit organe de commande comprend un interrupteur permettant d'actionner ledit ventilateur par jeu de came sur un contacteur et par rotation du couvercle lorsque le ou l'un desdits canaux de circulation d'air et le canal de passage d'air dudit organe de commande sont en communication,
- ledit interrupteur comprend un contacteur, configuré pour être actionné par jeu de came,
- ledit contacteur est configuré pour être actionné par un profil prévu sur une périphérie dudit couvercle,
- ledit contacteur est configuré pour être actionné de manière à faire fonctionner ledit ventilateur au moins, par exemple uniquement, lorsque le ou l'un desdits canaux de circulation d'air et le canal de passage d'air dudit organe de commande sont en communication,
- ledit ventilateur est muni d'une prise de raccordement d'alimentation à un réseau de bord d'un véhicule automobile,
- ledit ventilateur est muni d'une alimentation autonome dans le boîtier par piles, notamment par piles remplaçables par l'utilisateur ou batterie rechargeable par câble amovible
- la vitesse dudit ventilateur est gérée par ledit moyen de contrôle notamment sous la forme simplifiée d'une touche de fort débit..

Les but, objet et caractéristiques de la présente invention ainsi que ses avantages apparaîtront plus clairement à la lecture de la description ci-dessous des modes de réalisation préférés du dispositif de diffusion d'au moins un agent volatil selon l'invention, faite en référence aux dessins dans lesquels :
- la figure 1 montre en perspective, selon une vue éclatée et de façon schématique, un dispositif de diffusion, selon un mode de réalisation de la présente invention,
- la figure 2 représente, de façon schématique, une vue de dessus dudit dispositif,
- la figure 3 représente en perspective et de façon schématique, une variante de réalisation du dispositif de diffusion conforme à l'invention, installé dans son support,
- les figures 4a et 4b illustrent deux variantes de réalisation d'un dispositif de diffusion conforme à l'invention.

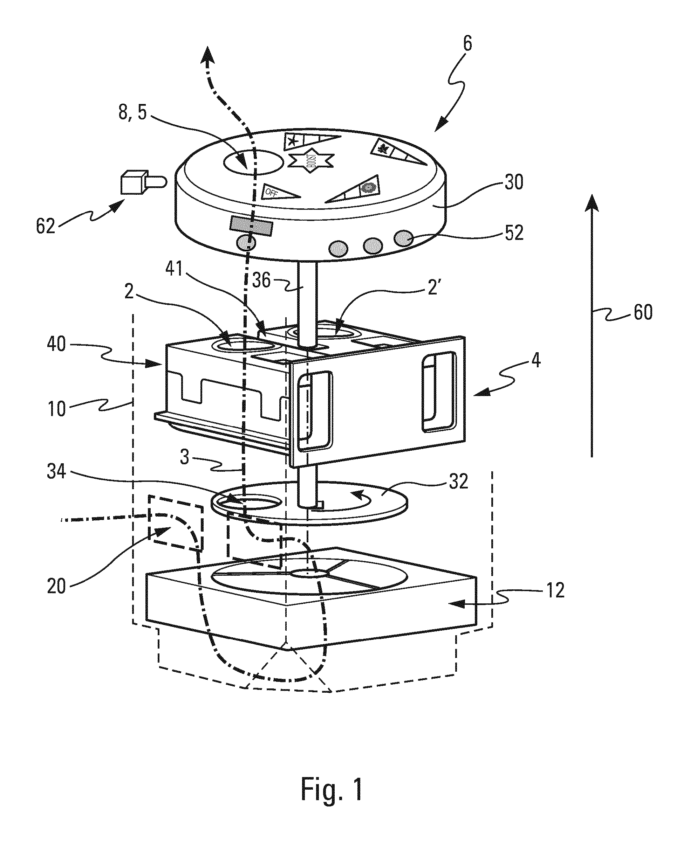

Comme illustré à la figure 1, l'invention concerne un dispositif de diffusion d'un ou plusieurs agents volatils. Elle trouvera en particulier ses applications dans l'équipement de l'habitacle des véhicules automobile, notamment comme équipement après-vente.

Ledit dispositif de diffusion comprend un ou plusieurs éléments de diffusion (non visibles) adaptés pour stocker et diffuser une quantité déterminée d'agents volatils, notamment une ou plusieurs fragrances en vue de parfumer l'air de l'habitacle du véhicule. Ledit dispositif est en outre pourvu d'un ou plusieurs canaux 2, 2' de circulation d'un flux d'air 3. Lesdits canaux de circulation 2, 2' sont adaptés pour que ledit flux d'air 3 se charge en agents volatils au contact desdits éléments de diffusion. Ledit dispositif comprend, par exemple, un réservoir 4, contenant le ou lesdits éléments de diffusion, le ou lesdits canaux de circulation 2, 2' traversant ledit réservoir 4. Autrement dit, le flux d'air se charge en agents volatils en traversant ledit réservoir 4.

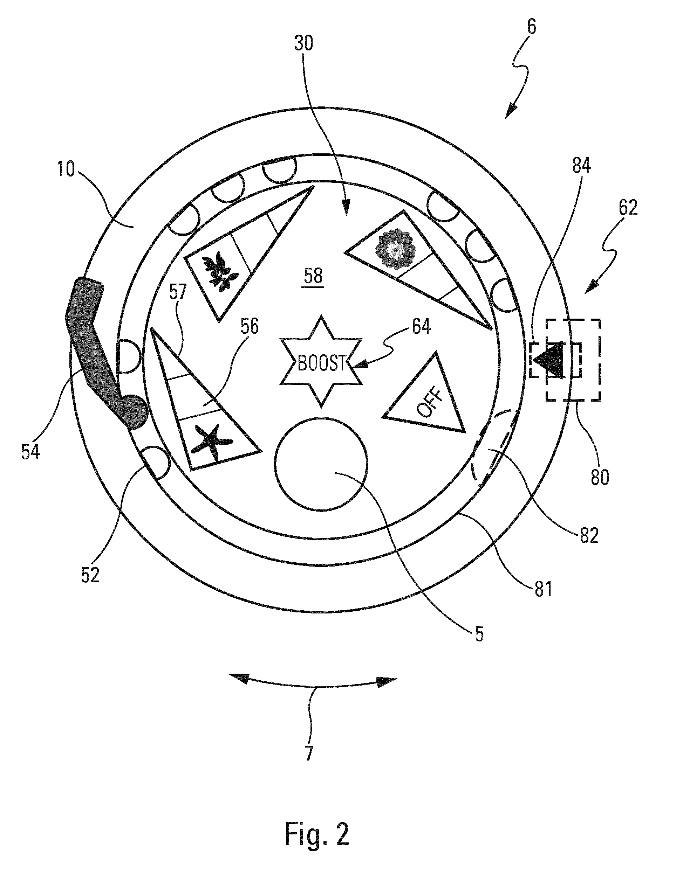

Comme cela est illustré à la figure 2, ledit dispositif comprend en outre un organe de commande 6. Ledit organe de commande 6 est actionnable manuellement par un utilisateur, selon la flèche repérée 7, et il est configuré pour prendre différentes positions sous l'action de ce dernier. Lesdites positions mettent ou non en communication le ou l'un desdits canaux de circulation d'air et un canal 5 de passage d'air dudit organe de commande 6 de manière à permettre la diffusion desdits agents volatils. C'est ainsi la position de l'organe de commande 6 qui détermine si un flux d'air, chargé ou non en agents volatils, est émis par ledit dispositif.

A la figure 1, la position de l'organe de commande 6 permet l'émission du flux d'air 3 en provenance de l'un 2 des canaux de circulation alors qu'il ferme un autre 2' des canaux de circulation.

Le dispositif de diffusion 1 comprend ici un boîtier 10 dont le contour est partiellement illustré, en pointillés. Ledit boîtier 10 est adapté pour recevoir en son sein différents composants, décrits ci-dessous. Le boîtier 10 est pourvu d'une ou plusieurs ouvertures 20 utilisées pour une entrée d'air afin de laisser pénétrer de l'air à l'intérieur du dispositif de diffusion. Dans un souci de simplification des dessins, seulement certaines des entrées 20 ont été illustrées (toujours en pointillés). Ledit dispositif comprend aussi une sortie d'air 8 pour laisser sortir l'air à l'extérieur dudit dispositif. Elle est ici située au niveau de l'organe de commande 6 à l'endroit où ledit conduit 5 de passage d'air débouche extérieurement.

Le dispositif de diffusion comprend en outre un ventilateur 12, ici disposé à l'intérieur du boîtier 10. Ledit ventilateur 12 est adapté pour générer ledit flux d'air 3. Ledit ventilateur 12 est avantageusement configuré pour faire circuler ledit flux d'air 3 depuis la ou les entrées d'air 20 jusqu'à la sortie d'air 8.

Ledit boîtier 10 pourra en outre accueillir le réservoir 4. Selon un mode de réalisation, le réservoir 4 est amovible par rapport au boîtier, notamment à travers une trappe prévue sur l'une des faces de celui-ci. Ainsi, le réservoir 4 peut être ôté et remplacé par un autre réservoir du même type, par exemple lorsque le contenu dudit réservoir 4 est épuisé, ou ledit réservoir 4 peut être rempli à l'aide d'une nouvelle quantité d'agents volatils identiques ou différents.

Préférentiellement, le réservoir 4 et/ou le ventilateur 12 sont disposés de sorte qu'une direction d'un flux d'air 3 circulant entre le ventilateur 12 et le réservoir 4 est alignée avec la direction du flux d'air circulant dans le ou les canaux 2, 2' de circulation du réservoir 4 et/ou dans le canal 5 de passage d'air dudit organe de commande. On limite de la sorte l'encombrement du dispositif. En particulier, les différents composants du boîtier 10, c'est-à-dire le ventilateur 12, le réservoir 4 et l'organe de commande 6 sont positionnés les uns au-dessus des autres suivant un axe d'empilement 60, afin d'obtenir un volume le plus réduit possible.

L'ordre des composants entre le ventilateur 12 et le réservoir 4 au sein de l'empilement peut varier selon l'utilisation souhaitée du dispositif de diffusion 1. Ainsi, de manière alternative au mode de réalisation illustré, le ventilateur 12 peut être positionné en aval du réservoir 4.

Afin d'obtenir une compacité minimale du dispositif de diffusion 1, la sortie du ventilateur 12 peut être au contact direct avec l'entrée du réservoir 4 quand le ventilateur 12 est positionné en amont du réservoir 4. De manière similaire, la sortie du réservoir 4 peut être au contact direct avec l'entrée du ventilateur 12 quand le réservoir 2 est positionné en amont du ventilateur 12.

L'empilement du ventilateur 12, du réservoir 4 et de l'organe de commande le long de l'axe d'empilement 60 permet d'obtenir un flux d'air dont la trajectoire est perpendiculaire par rapport aux plans dans lesquels se trouvent respectivement le réservoir 4 et le ventilateur 12. La sortie d'air 8 pourra être positionnée selon l'axe d'empilement 60 afin d'obtenir un flux d'air dont la trajectoire est sensiblement rectiligne par rapport au réservoir 4 et au ventilateur 12.

L'empilement du ventilateur 12, du réservoir 4, et de l'organe de commande 6 permet en outre de limiter les pertes de charge du flux d'air circulant au sein du dispositif de diffusion 1, notamment en aval du ventilateur 12. Ainsi, de manière avantageuse, l'utilisation du dispositif de diffusion 1 permet de diffuser de manière optimale un ou plusieurs agents volatils tout en limitant la puissance du ventilateur 12 nécessaire à la circulation d'un flux d'air dans le dispositif de diffusion 1.

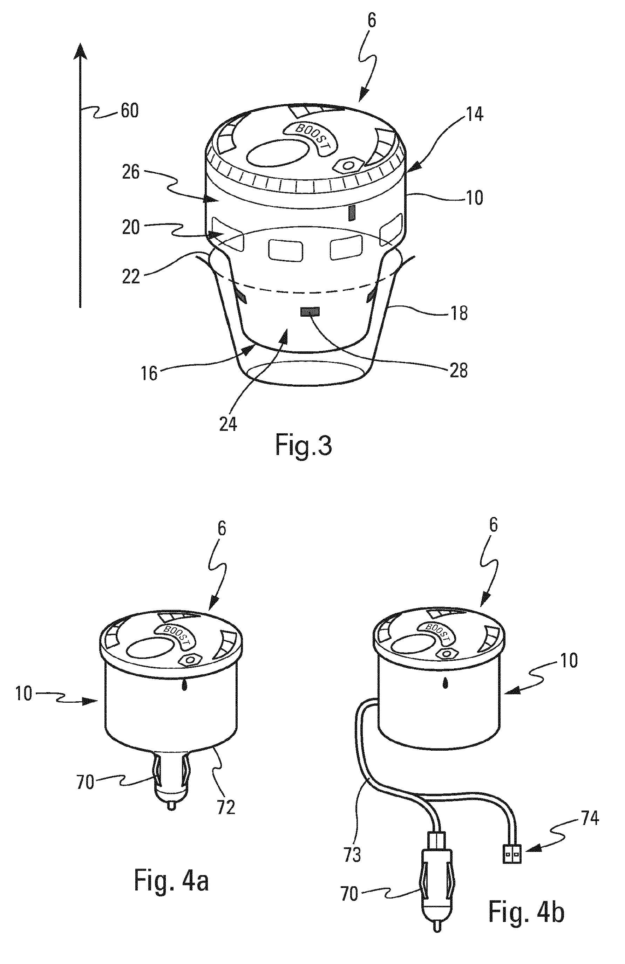

Comme cela est illustré à la figure 3, ledit boitier 10 est avantageusement fermé par ledit organe de commande 6. Ledit boîtier 10 présente plus précisément ici une ouverture situé à l'une 14 de ses extrémités longitudinale, ladite ouverture étant obturée par ledit organe de commande 6. Ledit organe de commande 6 est prévu mobile par rapport audit boîtier 10 pour occuper les différentes positions permettant de contrôler la diffusion. Ici, ledit organe de commande 6 est mobile par rapport audit boîtier 10 selon un mouvement de rotation autour d'un axe d'extension longitudinale dudit boîtier 10, c'est-à-dire un axe orienté suivant ladite direction d'empilement 60. Ledit boîtier 10 pourra de la sorte présenter une symétrie de révolution, notamment une forme sensiblement cylindrique de section circulaire.

Ledit boîtier 10 comprend avantageusement une extrémité 16, opposée à celle 14 accueillant l'organe de commande 6. Cette dernière extrémité 16 est apte à être insérée dans un support 18 dudit dispositif, ici un porte-gobelet. Lesdites entrées d'air 20 du dispositif sont alors positionnées de façon à être laissées libres une fois ledit dispositif inséré dans son support 18. Selon le mode de réalisation illustré, lesdites entrées d'air 20 sont situées au-dessus d'un bord supérieur 22 du support 18, voire affleurent au niveau dudit bord supérieur 22.

Une zone 24 accueillant ledit ventilateur 12 pourra être située au niveau de ladite extrémité 16 opposée à celle accueillant ledit organe de commande 6. Selon la direction d'empilement 60, la ou les entrées 20 sont alors disposés entre ladite zone 24 accueillant ledit ventilateur 12 et une zone 26 dudit boîtier 10 accueillant ledit réservoir 4.

Afin de sécuriser ledit boîtier 10 dans son support 18, ledit boîtier 10 pourra être muni d'ergots 28 de maintien, en particulier de blocage en rotation.

Si l'on se reporte de nouveau à la figure 1, comme déjà évoqué, le dispositif de diffusion comprend dans le mode de réalisation illustré une pluralité de canaux de circulation 2, 2' et l'organe de commande 6 remplit à la fois une fonction de réglage de débit et une fonction de sélection du ou des canaux de circulation parcouru par le flux d'air 3. Dans des versions simplifiées, il pourra aussi uniquement permettre un réglage de débit, voire une simple fermeture/ouverture, sans sélection de canaux.

L'organe de commande 6 comprend au moins un couvercle 30, disposé sur le réservoir 4. Ledit couvercle 30 est ici la partie de l'organe de commande 6 fermant ledit boîtier 10. Ledit couvercle 30 est pourvu dudit canal 5 de passage du flux d'air. L'organe de commande 6 comprend en outre au moins un disque 32 muni d'une ouverture 34, située en correspondance dudit canal 5 de passage du flux d'air. Ledit couvercle 30 et ledit disque 32 sont situés de part et d'autre dudit réservoir 4. On fiabilise de la sorte la sélection du canal de circulation du réservoir 4 parcouru par le flux d'air 3. Ledit organe de commande 6 comprend encore un axe d'entrainement 36, reliant ledit couvercle 30 et ledit disque 32 de sorte que ledit couvercle 30 et ledit disque 32 entrent simultanément en rotation et le canal de passage 5 reste aligné avec l'ouverture 34 dudit disque 32.

Le réservoir 4 permettant de stocker le ou les agents volatils pourra avoir la configuration suivante. Il comprend un corps 40 dans lequel une fente 41 est présente, ladite fente 41 permettant l'insertion dudit réservoir 4 à l'intérieur du dispositif de diffusion. La fente 41 est nécessaire afin de permettre le passage de l'axe de rotation 36 de l'organe de commande 6 au sein du réservoir 4. Le réservoir 4 comprend une ou plusieurs cavités, non visible. Dans le mode de réalisation illustrés, le réservoir 4 comprend une pluralité de cavités permettant de recevoir chacune le ou les éléments de diffusion, chacune correspondant à un canal de circulation du flux d'air. Le ou les éléments de diffusion se présentent, par exemple, sous la forme de sachets contenant une quantité déterminée de billes chargées en agents volatils.

Selon une première option, les agents volatils peuvent être différents afin de laisser l'utilisateur libre de choisir l'agent volatil qu'il préfère diffuser dans l'habitacle du véhicule automobile. Une deuxième possibilité consiste à imposer un agent volatil unique dans un premier sachet, le ou les autres sachets pouvant constituer un stock dudit agent volatil unique et éviter ainsi une pénurie en cas d'épuisement de l'agent volatil unique du premier sachet.

Selon un exemple particulier, le réservoir 4 comporte une ou plusieurs zones non pourvues d'éléments de diffusion. De telles zones sont associées en position au système, décrit plus bas, coupant l'alimentation du ventilateur, dès lors que l'utilisateur ne souhaite pas diffuser d'agent volatil au sein de l'habitacle.

Grâce à l'organe de commande, le canal de passage 5 et l'ouverture 34 du disque 32 sont positionnées pour permettre l'utilisation du dispositif de diffusion selon les souhaits de l'utilisateur. Si les cavités sont pourvues d'un agent volatil différent, l'utilisateur peut choisir le type d'agent volatil qu'il souhaite diffuser à l'intérieur de l'habitacle de son véhicule. Si l'utilisateur ne souhaite pas diffuser d'agent volatil à l'intérieur de l'habitacle de son véhicule, le canal de passage 5 et l'ouverture 34 du disque 32 sont alignées avec la zone dépourvue d'agent volatil.

Le couvercle 30 et le disque 32 sont respectivement positionnés à proximité de la surface inférieure et supérieure du réservoir 4, c'est-à-dire au-dessus et au-dessous du réservoir 4 et ce, afin d'assurer que ledit couvercle 30 et ledit disque 32 obturent, de façon hermétique, les différentes cavités. Ainsi, les cavités qui ne sont pas utilisées pour la diffusion d'un agent volatil sont obturées de façon optimale afin d'éviter l'évaporation des autres agents volatils stockées dans les autres cavités et la diffusion de ces autres agents volatils dans l'habitacle du véhicule automobile. Ainsi, lorsque l'agent volatil sélectionné par l'utilisateur correspond, par exemple, à l'agent volatil stocké au sein de l'une des cavités, l'organe de commande 6 est adapté pour obturer les autres cavités et laisser la première cavité ouverte. De manière similaire, lorsque l'utilisateur ne souhaite pas diffuser d'agent volatil au sein de l'habitacle du véhicule automobile, l'organe de commande 6 est adapté pour obturer la ou les cavités munis d'un agent volatil afin de laisser visible la zone dépourvue d'agent. Cette fonctionnalité présente un réel avantage. En effet, d'une part, le mélange des agents volatils, pendant la diffusion d'un agent volatil, est évité et, d'autre part, la diffusion d'un agent volatil à l'intérieur de l'habitacle du véhicule automobile, lorsque l'utilisateur ne souhaite aucune diffusion d'agent volatil, est empêchée.

Selon un mode de réalisation particulier, la position de l'organe de commande 6 peut être définie et utilisée en combinaison avec le flux d'air généré grâce au ventilateur 12 afin de déterminer une quantité précise d'agent volatil à diffuser à l'intérieur de l'habitacle du véhicule automobile.

L'organe de commande 6 peut être positionné de façon à laisser circuler le flux d'air à l'intérieur de l'une des cavités, en laissant le canal de circulation associé pleinement ouvert, et le ventilateur 12 peut être utilisé pour générer un flux plus ou moins important, générant ainsi une diffusion plus ou moins importante d'agent volatil stocké dans la cavité, à l'intérieur de l'habitacle du véhicule automobile.

De façon alternative, avec un flux d'air constant généré à l'aide du ventilateur 12, le canal de circulation associé à ladite cavité peut être plus ou moins obturé par ledit organe de commande 6, générant ainsi un flux d'air plus ou moins important circulant à travers ladite cavité. La quantité d'agent volatil diffusée est donc plus importante lorsque ledit canal de circulation est totalement ouvert.

Il convient de noter que la combinaison résultant de l'ouverture partielle d'un canal de circulation et d'un flux d'air généré grâce au ventilateur 12 permet de déterminer la quantité d'agent volatil diffusée à l'intérieur de l'habitacle d'un véhicule automobile.

Si l'on se reporte à la figure 2, on constate que, comme déjà évoqué, la sélection de la position de l'organe de commande a lieu manuellement. Il s'agit ici d'une position angulaire.

Dans le mode de réalisation illustré, le boîtier 10 et/ou ledit organe de commande 6 comprennent des éléments d'indexation visuelle et/ou mécanique permettant de repérer la position relative dudit organe de commande 6 par rapport au boîtier 10. Il s'agit, notamment, d'une part, de crans d'indexation 52 coopérant avec un excroissance articulée 54. Ici, les crans d'indexation 52 sont situés sur le pourtour du couvercle 30 et l'excroissance 54 sur une paroi intérieure d'une face latérale du boîtier 10. L'inverse est bien sûr possible. Il s'agit alternativement ou cumulativement, d'autre part, d'un indicateur visuel 56, situé par exemple au niveau d'une face visible 58 de l'organe de commande 6, ici au droit des crans d'indexation 52. L'indication visuel 56 pourra correspondre à la valeur du débit choisi.

Dans l'exemple, il est prévu trois série de trois crans d'indexation 52, chaque série correspondant à une ouverture plus ou moins grande de l'un des canaux de circulation. L'indicateur visuel 56 montre une rampe 57 symbolisant une croissance/décroissance du débit, en fonction du cran 52 dans lequel l'excroissance articulée 54 est engagée. Lesdites séries sont régulièrement réparties à la périphérie de l'organe de commande 6.

Autrement dit, la sélection de la série choisie correspond au canal de circulation qui sera plus ou moins ouvert et le choix du cran 52 d'indexation correspond au degré d'ouverture dudit canal. Un ou plusieurs crans supplémentaires, correspondant à une position de fermeture de tous les canaux de circulation, pourront également être prévus.

Ledit organe de commande 6 pourra en outre comprend un interrupteur 62 permettant d'actionner ledit ventilateur 12. Ledit interrupteur 62 comprend, par exemple, un contacteur 80, configuré pour être actionnée par jeu de came, ici par un profil prévu sur une périphérie 81 dudit couvercle. Plus précisément, il pourra s'agir d'une forme en creux 82. Lorsque ledit contacteur 80 se trouve en regard de ladite forme en creux 82, un doigt mobile 84 est dans une première position entraînant l'ouverture du contacteur et le ventilateur 12 n'est alors pas actionné. Lorsque le couvercle est déplacée en rotation, le doigt mobile quitte la forme en creux 82 et se trouve en position escamotée, comme cela est le cas sur la figure. Le contacteur est alors fermé et le ventilateur est actionné. Ladite forme en creux 82 est avantageusement positionnée en décalage axial par rapport aux crans d'indexation 52. Ici, un repère visuel « stop » est également prévu sur la surface 58 en regard de ladite forme en creux 82.

Selon une première variante, ladite forme en creux 82 comprend une dépression unique de faible envergure angulaire de sorte que le ventilateur est actionné dès que le couvercle 30 est tourné de quelques degrés. Selon un autre mode, non représenté, ladite forme en creux présente un profil permettant d'actionner ledit ventilateur uniquement lorsque le ou l'un desdits canaux de circulation d'air et le canal de passage d'air dudit organe de commande sont en communication, c'est-à-dire, ici, lorsque l'un des crans d'indexation coopère avec l'excroissance articulée.

L'organe de commande 6 pourra en outre comprendre des moyens de contrôle 64 comprenant notamment un circuit électronique permettant de gérer la vitesse du ventilateur 12, notamment sous la forme simplifiée d'une touche de fort débit. Ledit dispositif comprend en outre des pistes électriques, non illustrées, permettant d'établir un contact entre, d'une part, lesdits moyens de contrôle 64 et, d'autre part, ledit ventilateur 12 pour chacune des différentes positions dudit organe de commande 6.

Comme illustré aux figures 4a et 4b, ledit ventilateur 12 pourra être muni d'une prise 70 de raccordement à un réseau de bord d'un véhicule automobile. Il s'agit ici d'un connecteur électrique configuré pour être connecté, de manière amovible à un dispositif électrique disposé au sein dudit véhicule automobile, tel qu'un allume-cigare. Dans l'exemple illustré, ledit connecteur 70 est situé au niveau d'une face inférieure 72 du boîtier 10, située à proximité dudit ventilateur 12. En variante, un cordon 73 pourra relier ledit connecteur 70 et ledit ventilateur 12. En variante, ladite prise de raccordement 70 pour l'alimentation du ventilateur pourra être une prise 74 de type USB 5volts, l'équipement de certains véhicules étant désormais associé à ce nouveau standard.

## Revendications

1. Dispositif de diffusion comprenant un ou plusieurs éléments de diffusion adaptés pour stocker et diffuser une quantité déterminée d'agents volatils, ledit dispositif étant pourvu d'un ou plusieurs canaux (2, 2') de circulation d'un flux d'air adaptés pour que ledit flux d'air se charge en agents volatils au contact desdits éléments de diffusion, ledit dispositif comprenant en outre un organe de commande (6), actionnable manuellement par un utilisateur, ledit organe de commande (6) étant configuré pour prendre différentes positions sous l'action de l'utilisateur, lesdites positions mettant ou non en communication le ou l'un desdits canaux (2, 2') de circulation d'air et un canal (5) de passage d'air dudit organe de commande (6).

2. Dispositif de diffusion selon la revendication 1, ledit dispositif comprenant un ventilateur (12), apte à générer ledit flux d'air, et/ou un réservoir (4), contenant le ou lesdits éléments de diffusion, le ou lesdits canaux (2, 2') de circulation traversant ledit réservoir (4).

3. Dispositif de diffusion selon la revendication 2, dans lequel l'organe de commande, le réservoir (4) et/ou le ventilateur (12) sont disposés de sorte qu'une direction d'un flux d'air circulant entre le ventilateur (12) et le réservoir (4) est alignée avec la direction du flux d'air circulant dans le ou les canaux (2, 2') de circulation du réservoir (4) et/ou dans le canal (5) de passage d'air dudit organe de commande (6).

4. Dispositif de diffusion selon l'une quelconques des revendications 2 ou 3, dans lequel ledit organe de commande, ledit réservoir (4) et/ou ledit ventilateur (12) sont disposés l'un sur l'autre selon une direction d'empilement (60).

5. Dispositif de diffusion selon l'une quelconque des revendications 2 à 4 comprenant un boîtier (10) accueillant ledit ventilateur (12) et ledit réservoir (4), ledit boitier (10) étant fermé par ledit organe de commande (6), prévu mobile par rapport audit boîtier (10).

6. Dispositif de diffusion selon la revendication 5, dans lequel ledit boîtier (10) comprend une extrémité (16), apte à être insérée dans un support (18) dudit dispositif, ledit boîtier (10) comprenant en outre une ou des entrées d'air (20), positionnées de façon à être laissées libres une fois ledit dispositif inséré dans son support (18).

7. Dispositif de diffusion selon la revendication 6, dans lequel ledit ventilateur (12) est situé au niveau de ladite extrémité (16).

8. Dispositif de diffusion selon l'une quelconque des revendications 6 ou 7 dans lequel la ou les entrées d'air (20) sont disposées entre le ventilateur (12) et une zone (26) dudit boîtier (10) accueillant ledit réservoir (4).

9. Dispositif de diffusion selon l'une quelconque des revendications 5 à 8 dans lequel le boîtier (10) et/ou ledit organe de commande (6) comprennent des éléments d'indexation visuelle et/ou mécanique permettant de repérer la position relative dudit organe de commande (6) par rapport au boîtier (10).

10. Dispositif de diffusion selon l'une des revendications 2 à 9 dans lequel ledit organe de commande (6) est mobile en rotation autour d'un axe de rotation.

11. Dispositif de diffusion selon la revendication 10 dans lequel l'organe de commande (6) comprend au moins un couvercle (30) disposé sur le réservoir (4) et pourvu dudit canal (5) de passage du flux d'air.

12. Dispositif de diffusion selon la revendication 11 dans lequel l'organe de commande (6) comprend au moins un disque (32) muni d'une ouverture (34), située en correspondance dudit canal (5) de passage du flux d'air, ledit couvercle (30) et ledit disque (32) étant situés de part et d'autre dudit réservoir (4).

13. Dispositif de diffusion selon l'une des revendications 2 à 12, dans lequel ledit organe de commande (6) comprend un interrupteur (62) permettant d'actionner ledit ventilateur (12) par jeu de came sur contacteur (80) et rotation du couvercle (30) lorsque le ou l'un desdits canaux de circulation d'air et le canal de passage d'air dudit organe de commande sont en communication.

14. Dispositif de diffusion selon l'une quelconque des revendications 2 à 13 dans lequel ledit ventilateur (12) est muni d'une prise de raccordement (70) à un réseau de bord d'un véhicule automobile.

15. Dispositif de diffusion selon l'une quelconque des revendications 2 à 14, dans lequel le réservoir (4) est amovible.
